(19) **Europäisches Patentamt / European Patent Office / Office européen des brevets**

(11) **EP 2 114 246 B1**

(12) # EUROPEAN PATENT SPECIFICATION

(45) Date of publication and mention
of the grant of the patent:
**20.04.2022 Bulletin 2022/16**

(21) Application number: **08712764.3**

(22) Date of filing: **31.01.2008**

(51) International Patent Classification (IPC):
**A61B 5/083** (2006.01)   **G01N 33/497** (2006.01)
**G01N 21/35** (2014.01)   **A61B 5/08** (2006.01)
**A61B 5/097** (2006.01)   **B60K 28/06** (2006.01)
**A61B 5/00** (2006.01)   **A61B 5/087** (2006.01)
**G01N 21/3504** (2014.01)   **G01N 21/03** (2006.01)
**G01N 21/15** (2006.01)

(52) Cooperative Patent Classification (CPC):
**A61B 5/097; A61B 5/082; A61B 5/0836;**
**G01N 21/031; G01N 21/3504; G01N 33/4972;**
A61B 5/0878; B60K 28/063; G01N 21/15

(86) International application number:
**PCT/SE2008/050124**

(87) International publication number:
**WO 2008/108714 (12.09.2008 Gazette 2008/37)**

(54) **INTERACTIVE ALCOMETRY**

INTERAKTIVE ALCOMETRIE

ALCOOMÉTRIE INTERACTIVE

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **01.02.2007 SE 0700236**

(43) Date of publication of application:
**11.11.2009 Bulletin 2009/46**

(73) Proprietor: **Senseair AB**
**820 60 Delsbo (SE)**

(72) Inventor: **HÖK, Bertil**
**S-723 44 Västerås (SE)**

(74) Representative: **Barker Brettell Sweden AB**
**Östermalmsgatan 87B**
**114 59 Stockholm (SE)**

(56) References cited:
**EP-A2- 1 062 909        WO-A1-2007/046745**
**US-A- 4 749 553        US-A- 5 376 555**
**US-A- 5 531 225        US-A1- 2003 109 795**
**US-A1- 2004 141 171        US-A1- 2006 000 723**
**US-A1- 2006 153 740        US-A1- 2007 016 092**
**US-A1- 2008 061 238**

• **LAMBERT D.K. ET AL.: 'Passive Sensing of Driver
Intoxication' 2006 SAE WORLD CONGRESS,
DETROIT, MICHIGAN, (SAE TECHNICAL PAPER
SERIES), [Online] 03 April 2006 - 06 April 2006,
pages 1 - 12, XP008115164 Retrieved from the
Internet:
<URL:http://www.delphi.com/pdf/techpapers/2
006-01-1321.pdf>**

**Description**

The present invention relates to the determination of alcohol concentration in expired air, and addresses central aspects concerning measuring accuracy, response time, and interaction between the measuring object or operator on the one hand, and the measuring apparatus, on the other.

A number of methods and devices for alcometry, i e the determination of alcohol concentration in expired air, are described in the literature, see examples in the patent documents: US 2007/016092 A1, US 5 531 225 A, US 5 376 555 A, US 5 376 555 A, US 2004/141171 A1, US 2006/153740 A1, WO 2007/046745 A1, US 2008/061238 A1.

Several of these have rendered widespread use, both as measuring instruments, and as control devices. The alcohol interlock is one example of the latter, preventing vehicle drivers from starting the vehicle without an approved breath sample, i e with an alcohol concentration not exceeding the prevailing concentration limit.

Within alcometry, two measuring principles are dominating, making use of physical and chemical properties of alcohol. The latter category is essentially based on combustion mediated by a catalyst. The alcohol concentration can be determined by measuring the developed combustion energy, for example, in a fuel cell or a semiconductor sensor. This sensor type is advantageous in terms of simplicity in design, and peripherals, such as electronic circuitry. Additionally, the semiconductor sensors have small physical dimensions and can be produced at low cost.

The characteristics of the catalyst, and the actual combustion temperature are determining the selectivity of catalytic sensors. Since other organic substances are also being combusted in a similar manner, absolute specificity is difficult to obtain. Another difficult problem is related to the long term properties of catalyst. Influence from certain substances makes repeated calibrations necessary, and constitutes a risk for manipulation. Such substances, e g gases containing sulphur, are prevailing both in expired air and as air pollutants. Catalytic sensors are unfortunately afflicted with reliability problems which have not been solved in a satisfactory manner. These properties are being used by police for evidential purposes against drunk driving. Measuring low concentrations is, however, demanding in terms of precision, and the IR-based evidential instruments on the market are expensive.

Infrared (IR) spectroscopy represents a physical method of measurement which is not afflicted with the problems mentioned above. This method makes use of the specific "finger print" that gas-phase alcohol produces when illuminated by infrared light. The absorption spectre is due to resonant molecular vibrations, which are specific to the atomic bonds within the molecule. From this the specific properties of the absorption spectre can be deduced, and its associated high selectivity against other substances, and security against manipulation. Furthermore, the use of IR-based instruments requires expert knowledge.

The performance of alcometers is often noted with respect to accuracy over a certain range of measurement. For evidential instruments, accuracy of $\pm 5\%$ is frequently required, whereas $\pm 20\%$ is considered adequate for screening and similar purposes. Alcometers for the consumer market has lower accuracy. Among these and screening instruments, systematic error caused by the poorly controlled condition of the catalyst is dominating. For IR-based instruments, systematic errors can be minimised by a calibration procedure. The remaining error has the character of stochastic noise from the information carrying sensor signal or signals. The error and the noise level can be expressed as a statistic entity, e g the mean square of random variations over a specified bandwidth.

An important aspect of alcometry concerns alcohol determination in relation to recommended or legislated concentration limits, e g for vehicle driving. In Sweden, 0.02% blood alcohol concentration is prevailing as the upper limit for vehicle drivers, corresponding to approximately 0.1 mg/L in a sample of expired air. In a major part of the European Union, the corresponding limit is 0.05% and 0.25 mg/L, respectively.

At measurements referring to a certain concentration limit, the ratio of false positive and negative outputs is a relevant value of performance. High accuracy, i e small error, leads to small probability of false outputs, and vice versa. On the other hand, for this purpose, the accuracy is only interesting in an interval close to the concentration, and rather uninteresting outside this interval.

At police controls and road blocks, sobriety tests are being increasingly performed. The Swedish police force is annually performing close to two million sobriety tests on vehicle drivers, and approximately 15 000 drunk drivers are caught this way. Negative outputs are thus being found in more than 99% of the test occasions. There seems to be a relation between the number of tests performed per unit of time, and the percentage of intoxicated drivers, as well as the number of alcohol related accidents per unit of time. In certain countries the percentage of drunk drivers can be higher than in Sweden, but there is a growing support for sober vehicle driving in many countries. The use of alcohol interlocks have also been successful, not only for conditional withdrawal of driver's licence after conviction for drunk driving, but also for quality assurance of transport services.

There are many situations and conditions outside the area of traffic safety, when testing of the sobriety of a person could be motivated. The practice of professions requiring precision, good judgement, dependence on certain individuals could be good reason for sobriety test. Moreover, at events of different kinds, there may be reason for sobriety test, e g at passage control stations.

Unfortunately, many application areas of alcohol determination have been hindered by practical and economic limitations of the present technology and engineering solutions. Besides the already mentioned, there are practical problems related to the actual sampling technique. State of the art solutions require exchangeable and disposable mouthpieces for hygienic reasons. Changing mouthpieces at each measurement is both time consuming, and adds costs. The total time lapse for one test could be several minutes at cold weather, which is unacceptable, e g at passage control, or in a vehicle integrated alcohol interlock.

Summing up, it may be concluded that state of the art alcometry is afflicted with a plurality of problems, touching upon central issues related to accuracy, time lapse, accessibility, reliability, and cost.

The object of the present invention is to solve the problems mentioned above, and related ones. The invention, a novel method and apparatus for the determination of alcohol concentration in expired air, exhibits significant advantages compared to the present state of the art. First, accuracy and time lapse can be adapted to specific requirements at each measuring occasion. Second, considerable cost savings are being achieved, since both cost of material and time lapse for each measurement can be considerably reduced. Third, very high reliability is achieved by avoiding the aging phenomena of e g catalytic sensors. Fourth, the need for maintenance of the apparatus is reduced, since it does not require repeated calibration. This also reduces the cost of maintenance. Fifth, the accessibility of the technique is improved for large groups of users. Sixth, the implementation of the apparatus at low production cost makes its price accessible to larger groups of users.

The characteristics of the method and the apparatus according to the invention are described in the enclosed claims. Below follows a comprehensive description of the basic elements of the method and the apparatus.

Determination of alcohol concentration of breath samples is preferably performed, according to the invention, primarily for hygienic reasons, by sampling without physical contact between the apparatus, and the respiratory organs of the test object. The test object or person is frequently a conscious individual, whose eventual intoxication is the object of investigation. The invention is, however, not limited neither with respect to test objects or detected substance. They are applicable also to higher animal species, and unconscious people, and also to other volatile substances than alcohol.

The breath sample is delivered by the test person by blowing towards a sensor arrangement positioned at 10-30 cm distance from the mouth and nose. Consequently, the breath sample is diluted with ambient air, requiring compensation for this dilution in order to make determination of alcohol concentration of the expired air possible. The relation between the externally measured alcohol concentration $C_{ext}$ and the alveolar $C_{alv}$ can simply be expressed by equation (1):

$$C_{ext} = D \cdot C_{alv} \qquad (1)$$

The variable D expresses the degree of dilution. For undiluted expired air, D=1, and at a very high degree of dilution, D→0.

Equation (2) concerns a relation which makes it possible to determine the degree of dilution D in real time, simultaneously with the corresponding measurement of alcohol concentration is performed, at essentially the same point. It is assumed that there is a measuring entity X which is exposed to the same dilution process as $C_{ext}$, $C_{alv}$. The expression for D is:

$$D = \frac{X_{ext} - X_{amb}}{X_{alv} - X_{amb}} \qquad (2)$$

With $X_{amb}$ is meant the measured value taken by the entity X in the ambient of the test object, whereas $X_{alv}$ is the corresponding measuring value. Examples of entities that can be considered to fulfil the criteria of the assumption are temperature, and concentrations of water vapour and carbon dioxide, respectively.

In the table below typical values are given of the measuring values of these entities in indoor environment:

| | Temperature (°C) | Water vapour concentration (mg/l) | Carbon dioxide concentration (% by volume) |
| --- | --- | --- | --- |
| $X_{amb}$ | 18-28 | 4-20 | <0.1 |
| $X_{alv}$ | 37 | 44 | 5.3 |

By measurement of $X_{ext}$ and $X_{amb}$, and insertion of $X_{alv}$ the value of which could be considered constant, D can be determined. The table indicates that $CO_2$ concentration has two significant advantages compared to the others. One is that the ambient concentration is very small compared to the alveolar. Thereby the influence from the ambient is at a minimum. Another advantage is that a detected difference in $CO_2$ concentration is most likely to have alveolar origin. On the other hand, the alveolar $CO_2$ concentration exhibits a somewhat larger variability than temperature and water vapour concentration. Temperature and humidity measurements have advantages with respect to simplicity, speed and cost. To achieve maximum security it is of course possible to use a combination of more than one measuring entity.

It should be noted that the anatomical and physiological deadspace can also cause dilution of the expired air. The anatomical deadspace includes the upper airways, and is approximately 150 ml for a normal adult person. Expired air from the anatomical deadspace is little mixed with alveolar air. The physiological deadspace is dependent on which substance it refers to, and is influences by e g the solubility of the substance within the mucosa. Taking these differences into account, the reasoning above can also be applied to sampling with a mouthpiece.

Measurement of temperature, concentration of water vapour and carbon dioxide is preferably performed by means of sensors specific to each entity. Temperature can be measured with resistive sensors or thermo elements, both providing adequate accuracy. In order to obtain necessary speed of response, 0.5 seconds or shorter, small physical dimensions and therefore small thermal mass is required. Miniaturised temperature sensors with adequate response time are commercially available. The same holds for the measurement of water vapour concentration and carbon dioxide. The first case deals with a capacitive measuring principle, making use of the high dielectric permittivity of water. For $CO_2$ determination IR absorption can be used, and thereby be integrated with alcohol determination as described above. $CO_2$ exhibits specific absorption in a narrow wavelength band around 4,26 $\mu$m, whereas alcohol has absorption peaks at 3.4 and 9,5 $\mu$m. Water vapour exhibits a relatively broad absorption band at 2,6-2,8 $\mu$m.

In order to fulfil the criteria of equation (1) and (2) it is required that the sensors for alcohol and entities X are measured at the same point, setting demands on the size and positioning of the sensors. Alternatively, the air sampling part of the apparatus is supplied with a tubing line, and a pumping device for transport of the breath sample from the actual sampling point to the physical position of the sensors.

By insertion of current values of D and $C_{amb}$ it is easy from equation (1) to determine $C_{alv}$. Experiments have shown that it is possible to determine alcohol concentration without physical contact at a distance of up to 30 cm between the mouth/nose of a test person and the sampling point. This assumes, however, the active participation of the test person to blow expired air towards the sensor. Passive measurement without the cooperation of the test person requires a shorter measuring distance, up to 10 cm, since only resting respiration can be assumed.

The inaccuracy IA of the method is determined by the measurement error $C_{error}$ in relation to the current measured value $C_{alv}$, and can suitably bed described by the ratio between these. $C_{error}$ can in its turn be partitioned into relatively constant factors and such that can be influenced from one occasion to another, and even during ongoing measurement. The result of such partitioning is shown in equation (3):

$$IA = \frac{C_{error}}{C_{alv}} = \frac{C_{resolution}}{C_{alv} \cdot D \cdot \sqrt{\Delta t}} \qquad (3)$$

$C_{resolution}$ refers to the remaining resolution of the instrument which is difficult to control, and limited by noise sources of fundamental origin, or other random factors. Systematic error sources are assumed to be eliminated by a sufficiently accurate calibration procedure. $C_{resolution}$ is connotated in the dimension of the measurand, in this case alcohol concentration, divided by the square root of the current bandwidth, $\Delta f$, $\sqrt{(Hz)^{-1}}$.

The entities $C_{alv}$ and D of equation (3) are assumed to be measured values accumulated during a certain measuring time $\Delta t$. Prolongation of this time can therefore be considered identical to a reduction of bandwidth.

Equation (3) illustrates the possibility to influence the inaccuracy either by the degree of dilution D or by the measuring time $\Delta t$. In order to increase the accuracy a certain factor from a given starting point, it is either required to increase D by the corresponding factor, or by increasing the measuring time the square of this factor, or a combination of these measures.

In the method according to the invention, the relation of equation (3) is used in each single determination of alcohol concentration to adapt measuring time and accuracy to the object or requirement of that occasion. The adaptation is performed interactively between the measuring apparatus and the user/test person or operator. More specifically, the apparatus leaves in real time, i e without a limiting time delay, indication of current or accumulative measuring value and error or thereto related entity. The factor D can be such entity.

The user, test person or operator can choose at any instant whether to finish the determination, or continue in order to increase accuracy. Such increase is possible according to equation (3) either by prolonging the measuring time, or by changing the expiration of the test person in relation to the apparatus, which brings about an increase of the dilution factor D, and a corresponding increase of accuracy. D is also influenced by the position of the sensors with respect to the test person. An operator can, guided by the indicated accuracy, move the sampling point with respect to the respiratory organs of the test person until acceptable level is obtained. Measurement in real time means that the procedure is possible at the test person's normal respiratory activity, without active cooperation from his/her part. It is therefore possible to perform measurement even in unconscious people, and in animals.

The method according to the invention thus makes possible the interactive control of the accuracy and time lapse of alcohol determination. The relations (1), (2), and (3) are being employed for computation of instant values of $C_{alv}$, and D in real time during ongoing measurement, as soon as a significant difference $X_{ext}$-$X_{amb}$ occur, apart from normally occurring random variations. A starting criterion for the computation of $C_{alv}$ and $C_{error}$ is that D is above a certain minimum value, $D_{min}$. The accuracy of the first determination after reaching the threshold $D_{min}$ is by definition low, but can rapidly be improved, as higher values of D are obtained.

If the current issue is limited to determine whether the alcohol concentration exceeds a certain concentration limit $C_{limit}$ or not, the method according to the invention is offering particular advantages. If the current measuring value $C_{alv}$ with addition of the error $C_{error}$ is lower than $C_{limit}$, i e if

$$C_{alv} < C_{\lim it} - C_{error} \qquad (4)$$

The determination can be finished directly without trying to improve accuracy by prolonged measuring time or adjustment of sensor positions etc. Correspondingly, the same procedure may be adopted when the instant measured value of alcohol concentration exceeds the limit value added to the error:

$$C_{alv} > C_{\lim it} + C_{error} \qquad (5)$$

However, if

$$C_{\lim it} - C_{error} \le C_{alv} \le C_{\lim it} + C_{error} \qquad (6)$$

then accumulation of instant values should continue in order to reduce the error, with the objective of true knowledge whether the concentration limit is exceeded or not.

The case (4) is valid for more than 99% of randomly selected Swedish vehicle drivers, according to the description above. The method according to the invention should, for this group mean significantly reduced measuring time, and therefore lower cost per sample.

The invention is described in more detail in connection with the enclosed figures 1-4. Figure 1 shows a flow chart of the method according to the invention. Figure 2 schematically shows the timing of events during a determination of alcohol according to the invention. Figure 3 shows examples of accuracy for different applications. Figure 4 schematically shows one embodiment of the apparatus according to the invention.

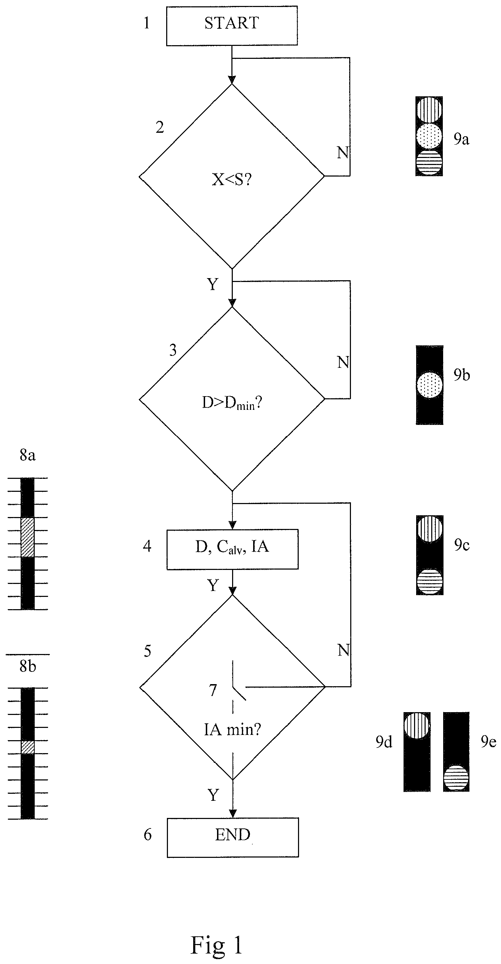

The flow chart of figure 1 comprises a number of steps corresponding to the conditions of the apparatus. To start with, at the activation of the apparatus a starting position 1 occurs, and is directly transferred into a waiting mode 2. The activation can be performed manually by an on/off button, or automatically from some other equipment connected to the apparatus. During the waiting mode 2, a stability test is performed of the sensor signals corresponding to alcohol concentration and degree of dilution, respectively. If the sensor signals are being interfered in any way, the waiting process is continuing until stability has been reached.

When the criterion is fulfilled that the variation in time of the abovementioned signals does not exceed a certain threshold S, the apparatus is transferred into the measuring mode 3. In this mode the test person can be instructed to exhale towards the sensors of the apparatus, alternatively, these are positioned without the active cooperation of the test person to the vicinity of his/her respiratory organs. Analysis of the sensor signals corresponding to dilution is being performed continuously, and when the criterion for the dilution factor $D>D_{min}$ has been reached, the apparatus is being transferred into a computational mode 4. In this mode, measurement and computing of the entities D, $C_{alv}$ and IA are being performed simultaneaously in real time, and current values of the at least the latter are being communicated, likewise in real time, to the operator, the user or the test person.

During the condition of measuring and computation 4, the operator, user or test person can choose to finish the measurement if sufficient accuracy has already been obtained. Finishing is done by the test person finishing to blow expired air towards the sensors of the apparatus, or by withdrawing these from the vicinity of the respiratory organs. The operator, user or test person can also choose to continue blowing expired air towards the sensors in order to accumulate further measuring points, or eventually increasing the dilution factor D in order to increase accuracy.

The task of carrying out measurements and computations simultaneously in real time sets special demands on the apparatus according to the invention. On the one hand, signals from sensors must be sampled and put in a buffer memory, and from there be transferred to a arithmetic-logic computation unit in which the equations (1), (2), and (3), or corresponding ones, are being executed. The result from the computations, as well as eventual interim results, should also be stored in a buffer memory, and from there be visualised in order to be perceived by the test person or operator.

The time interval between each indication should not exceed 0.5 seconds in order for the test person or operator to perceive the indicated information as instantaneous. With commercially available microprocessors, including both buffer memory and arithmetic-logic unit, it is possible to fulfil this requirement. Preferably, the microprocessor also includes permanent memory for storing the program controlling computations and data communication.

The change in time of the measurement error is being computed and indicated in real time, clearly visible for the test person or operator during the subsequent phase 5. When the accumulated inaccuracy IA reached a lowest value, automatic transfer to a final condition 6 can take place, whereby the accumulated values of $C_{alv}$ and $C_{error}$, respectively, may be valid as final for the current determination. The instruction of the operator, user or test person concerning the eventual continuation of the measurement or its termination, is however overruling this automation, and may be controlled by a switch position 7 controlled by this person. It is, for example, possible to continue measurement during one or several subsequent expirations.

The various conditions 1-6 are indicated by the apparatus according to the invention by audiovisual or haptic arrangements. For example, the measured value and inaccuracy may be indicated as position and width on a measuring scale 8a, 8b, in which 8a is indicating a relatively large measurement error, whereas 8b indicates essentially the same measuring value but a smaller error.

An alternative embodiment of indication of conditions is shown in the sequence 9a-9e, using symbols of traffic lights. The start- and wait conditions 1 and 2 are indicated in 9a by illumination from red, yellow and green lamps. The measuring condition 3 is indicated by illumination from the yellow lamp 9b, eventually flashing in order to call attention. The condition for measurement and computation 4 is indicated by illumination from both the red and green lamp, 9c. The green and red lamp emits different intensities depending on the current magnitude of the measuring value compared to a concentration limit, using the relations (4), (5), and (6). In the final condition (6) the inaccuracy is in most cases so small that a definite response can be provided, as to whether the concentration limit is exceeded or not. In such case it gives rise to a red stop signal 9d if exceeded, and a green light 9e if not.

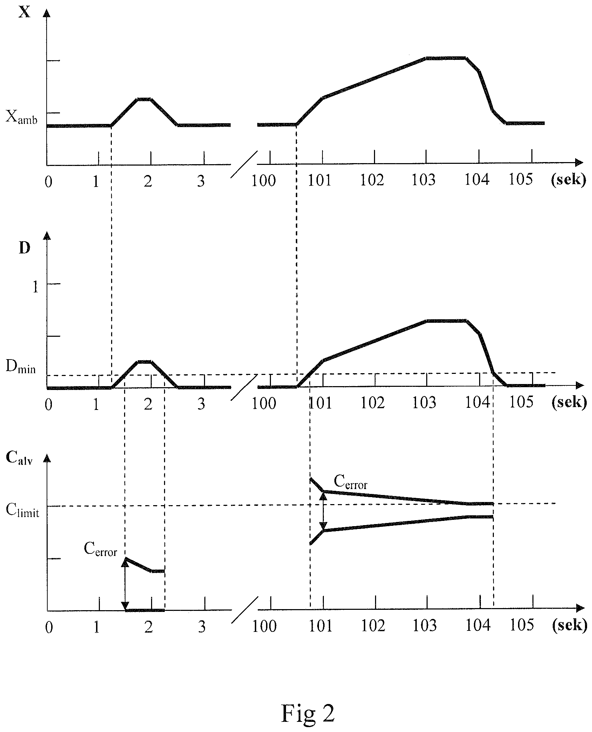

Figure 2 schematically shows typical time sequences of the sensor signal X, and the degree of dilution D deduced from equation (2), and the computed alveolar alcohol concentration, with computed from equations (1) and (3), with accumulated measurement error inserted into the figure as lower and upper limit for the measuring value. The variation as a function of time is shown for the variables X (upper graph), D (middle graph) and $C_{alv}$, $C_{error}$ (lowest graph) partly for a sober test person (time scale 0-3 sec) and partly for a person with alcohol concentration slightly below the concentration limit $C_{limit}$ (time scale 100-105 sec).

From a stable initial value $X_{amb}$ corresponding to that valid for the ambient, X will continuously rise to a higher value as long as the test person is blowing towards the sensor arrangement. When expired air from the test person has hit the sensor arrangement a signal plateau is reached and maintained as long as the stream of air is maintained, i e at the duration of the expiration. After that the level is decreasing, and retains the initial value $X_{amb}$.

The variable D varies in time in an identical way as the variable X, which is obvious from equation (2). This is illustrated in the middle curve of figure 2. Experiments have shown that D=0,3-0,5 could be a realistic level, obtainable by forced expiration towards a sensor arrangement at a distance of 10-30 cm. This level is, however, obtained after one, or a few seconds of blowing. At an earlier point in time, the abovementioned level $D_{min}$ is reached, at which the apparatus switches to the measuring and computational condition. A suitable value could be $D_{min}$=0,1...0,2. It is first at the point in time when $D_{min}$ is exceeded that measured values of alcohol concentration appears which together with the measurement error is illustrated on the lower curve. In the left series of curves, with a sober test person, immediate response that the level is below the concentration limit is provided, and therefore the test person can choose to finish the determination.

In the series of curves at right it is from the beginning not clear whether the concentration limit will be exceeded or not, since this limit falls within the tolerance of the measuring value. The test person therefore chooses to continue the determination, whereby the measurement error decreases. The accumulated measuring error approaches a minimum at the end of the breath.

As illustrated in fig 2 the method according to the invention allows for significantly reduced time for measurement and computation when a sober person is subjected to a sobriety test with respect to a given concentration limit.

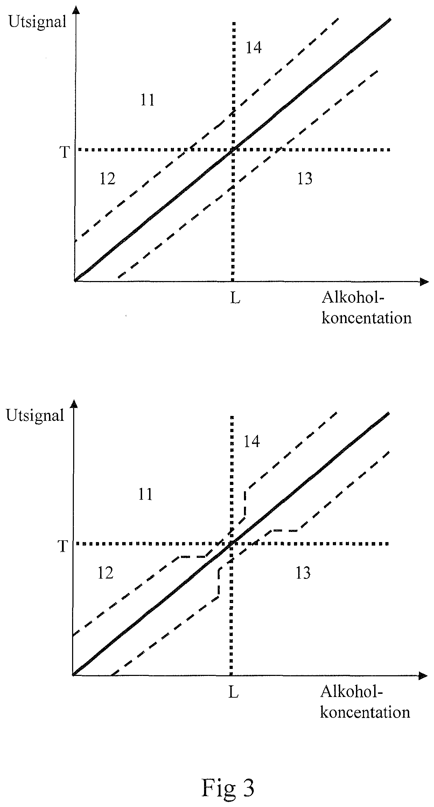

Figure 3 shows two diagrams of which the upper concerns a measuring instrument according to the present state of the art, whereas the lower provides an example of the present invention. In both diagrams the output signal (solid lines) and measurement error (dashed lines) are shown as a function of alcohol concentration. Moreover, a concentration limit L is indicated on the axis of alcohol concentration, and a trigger level T on the axis of the output signal. Thereby four quadrants 11, 12, 13, 14 within the diagram are marked. The quadrants 12 and 14 represent truly negative and positive outputs of the apparatus, whereas the quadrants 11 and 13 represent false positive and negative outputs, respectively

In a conventional apparatus the measurement error cannot be influenced by the user, implicating a risk for false outputs according to the upper diagram. According to the present invention, however, it is possible to adapt the measurement error to minimise it at about the concentration limit L and the trigger level T. As already described this adaptation is performed between the user, operator or test person on the one hand, and the apparatus on the other.

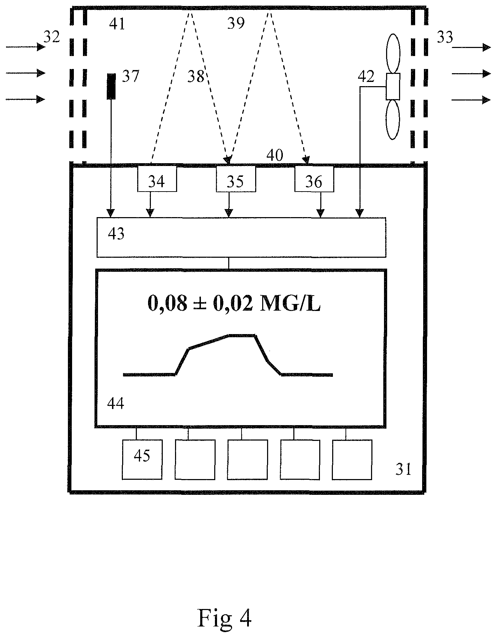

Figure 4 shows schematically the design of the apparatus according to the invention according to a preferred embodiment. The apparatus is preferably embedded in a apparatus box or housing 31, the size of which is adapted to handheld use or integration within vehicle instrumentation, e g a vehicle steering wheel. The physical dimensions of the housing 31 should not exceed 120 x 120 x 30 mm. The housing 31 is supplied with openings 32, 33 for in- and outflow of breath sample. Thereby a measuring cell 41 is defined and being trans-illuminated by collimated infrared light from a source 34. The IR beam 38 is reflected a few times against the reflecting surfaces 39, 40 within the measuring cell 41, before it hits the detectors 35, 36 which are adapted to selectively detect radiation within the wavelength bands within which alcohol on the one hand, and water vapour, carbon dioxide on the other exhibits substance specific absorption.

A temperature or flow sensor 37 with response time 0.5 seconds or shorter, is located near the entrance 32 of the measuring cell 41. The sensor 37 can also be combined with an electric heating device in order to heat the incoming breath sample. The purpose may partly be to measure flow velocity according to the principle of hot wire anemometry, and partly to prevent condensation due to the humidity of expired air. Further there is a device 42 for active air flow through the measuring cell 41. This device can for example be a miniature fan or pump.

The openings 32 and 33 to the measuring cell 41 are in figure 4 schematically drawn as double gratings for two reasons. Partly the gratings themselves are protecting against dirtying of the sensitive optical elements within the measuring cell 41 and can be supplied with different types of filter for use in particularly harsh environment. Partly double and deflectable gratings which are only opened a short while during measurement provide an effective protection between the measurement occasions.

The electric signals generated by the sensors 35, 36, 37 are subject to amplification, filtering and other signal processing within the electronic unit 43, which also executes control and drive of the IR source 34, the heating device within the sensor 37 and the flow device 42. The electronic unit 43 preferably includes a microprocessor or corresponding, for execution of a program stored in a memory, including the computations and condition indications described above.

The IR source 34 is preferably modulated at a frequency of 2 Hz or higher. Thereby problems with offset drift of the IR detector and input amplifier are eliminated, while obtaining adequate response time. The demands on response time are primarily defined by real time visualisation of measuring values, being perceived as instantaneous by the operator or test person. In practice this corresponds to a response time of 0,5 seconds or less.

The electronic unit 43 preferably includes circuits for digital signal communication to an integrated presentation unit 44 for indication of measuring values and to other units. The presentation unit 44 is in the most simple case a small number of lamps or light emitting diodes as described in conjunction with figure 1, but could also be a graphic and/or alphanumeric display as illustrated in figure 4, for more detailed indication of the variation in time of the sensor signals. The control of the user or operator on the electronic unit 43 is exercised by manual control devices 45. The supply voltage could either be taken from a built-in battery or from an external voltage source.

The apparatus according to the invention is designed with respect to choice of materials and components, for maintenance-free operation over at least 15 years or 30 000 measuring occasions.

The apparatus according to the invention is designed so that a normally talented person without trouble can use it. The described interaction, including the choice of finishing point for the determination has an intuitive character and therefore demands a minimum of previous instruction.

It should further be noted that the schematically illustrated apparatus of figure 4 is built with materials and components which can be manufactured serially at low cost. Moreover the design of the apparatus is adapted to automatic assembly which means that the number of manual fabrication procedures is minimal. Also calibration, test and quality assurance can be effectively performed with automatic means. Conclusively, this implicates that the apparatus according to the invention can be given an attractive price, and thereby reach large groups of users. Elimination of periodic calibration during the life time of the product also contributes to provide the method and apparatus according to the invention low drift cost and high accessibility.

The embodiments of figure 1-4 are not as already pointed out not limiting for the applicability of the present invention. Its characteristics are defined by the enclosed claims.

## Claims

1. Method for the determination of alcohol concentration in expired air from a breath sample delivered by a test person blowing towards a sensor arrangement in an apparatus for alcohol concentration determination, by contactless sampling of said expired air wherein the breath sample is diluted with ambient air, comprising interaction between the apparatus on the one hand and the test person or an operator on the other, wherein

said interaction comprises a presenting unit within the apparatus presenting a measuring value and a measuring error of said determination to said test person or operator in real time during ongoing measurement;

said measuring error is dependent on the dilution of said expired air, accumulated time of said determination, and noise, interference or other random variations of said measuring value, said dilution being determined by measurement of temperature, concentration of water vapour, or carbon dioxide, or a combination of these entities, wherein

an adaptation of accuracy and time lapse of said determination to current objective or requirement is performed interactively between the apparatus and the test person or operator in that the test person or operator choose to finish the determination or continue the determination, wherein time lapse refers to the time required to perform the determination and wherein the prolonged measuring time and compensation for possible dilution of said expired air, is for the purpose of increasing said accuracy, and wherein

the presentation unit provides an indication of the measuring value and the measuring error by means of audiovisual or haptic indication from a presentation unit within said apparatus,

the method comprising a stepwise procedure, including a starting step, at which said apparatus is activated, followed by a waiting step at which sensor signals corresponding to said measuring value are controlled with respect to stability, subsequently followed by a step of measuring and computation at which real time alveolar alcohol concentration of alcohol and measuring error are carried out and visualised, wherein the real time alveolar alcohol concentration of alcohol and the measuring error are measured values accumulated during a certain measuring time and a final step following from an accumulated inaccuracy having reached a minimum, the accumulated inaccuracy being a ratio between the accumulated measuring error and the accumulated real time alveolar alcohol concentration of alcohol.

2. Method according to claim 1, wherein said visualisation has symbolic or numerical character, and being directed towards said test person or operator.

3. Method according to claim 1, wherein said alcohol determination is based on physical property, in particular being infrared absorption, and that said measuring error after minimising, essentially has stochastic character.

4. Apparatus for the determination of alcohol concentration according to the method of claim 1, comprising at least

a first sensor, the output signal of which is a monotonous function of said alcohol concentration,

a second sensor, the output signal of which is a monotonous function of the dilution of said expired air, whereby the response time of said first and second sensor does not exceed 0,5 seconds,

an electronic unit including a microprocessor, for execution of a program stored in a memory for processing said signals generated by said sensors including computation of the measuring value, the measuring error and said determination of alcohol concentration,

a presentation unit for audiovisual or haptic visualisation of said determination, measuring value in real time during ongoing measurement, wherein the apparatus is arranged to perform a stepwise procedure, including a starting condition, at which said apparatus being activated, followed by a waiting condition at which sensor signals corresponding to said measuring value being controlled with respect to stability, subsequently followed by a condition of measuring and computation at which real time alveolar alcohol concentration of alcohol and measuring error being carried out and visualised, wherein the real time alveolar alcohol concentration of alcohol and the measuring error are measured values accumulated during a certain measuring time and a final condition following from an accumulated inaccuracy having reached a minimum, the accumulated inaccuracy being a ratio between the accumulated measuring error and the accumulated real time alveolar alcohol concentration of alcohol.

5. Apparatus according to claim 4, wherein said apparatus is embedded in housing with outer dimensions not exceeding 120 x 120 x 30 mm, including device for controlled opening of said housing for receiving said expired air.

6. Apparatus according to claim 4, comprising at least one sensor for the measurement of said temperature or flow velocity of said expired air, whereby the response time of said sensor not exceeding 0,5 seconds.

7. Apparatus according to claim 4, comprising a measuring cell with opening for reception of said expired air said measuring cell being trans-illuminated by collimated infrared radiation, and means for active flow.

**Patentansprüche**

1.  Verfahren zur Bestimmung der Alkoholkonzentration in ausgeatmeter Luft aus einer Atemprobe, die von einer Testperson abgegeben wird, die in Richtung einer Sensoranordnung in einer Einrichtung zur Bestimmung der Alkoholkonzentration bläst, durch kontaktlose Probenahme der ausgeatmeten Luft, wobei die Atemprobe mit Umgebungsluft verdünnt wird, umfassend die Interaktion zwischen der Einrichtung einerseits und der Testperson oder einem Bediener andererseits, wobei

    die Interaktion eine Präsentationseinheit innerhalb der Einrichtung umfasst, die der Testperson oder dem Bediener während der laufenden Messung einen Messwert und einen Messfehler der Bestimmung in Echtzeit präsentiert;

    der Messfehler von der Verdünnung der ausgeatmeten Luft, der akkumulierten Zeit der Bestimmung sowie dem Rauschen, der Interferenz und anderen Zufallsvariationen des Messwerts abhängt, wobei die Verdünnung durch Messung der Temperatur, der Konzentration von Wasserdampf oder Kohlendioxid oder einer Kombination dieser Entitäten bestimmt wird, wobei

    zwischen der Einrichtung und der Testperson oder dem Bediener interaktiv eine Anpassung der Genauigkeit und der Zeitspanne der Bestimmung an das aktuelle Ziel oder die aktuelle Anforderung durchgeführt wird, indem die Testperson oder der Bediener entscheidet, die Bestimmung zu beenden oder die Bestimmung fortzusetzen, wobei sich die Zeitspanne auf die Zeit bezieht, die erforderlich ist, um die Bestimmung durchzuführen, und wobei die verlängerte Messzeit und der Ausgleich für eine mögliche Verdünnung der ausgeatmeten Luft dem Zweck der Erhöhung der Genauigkeit dienen, und wobei

    die Präsentationseinheit eine Anzeige des Messwert und des Messfehlers mittels einer audiovisuellen oder haptischen Anzeige von einer Präsentationseinheit innerhalb der Einrichtung bereitstellt,

    wobei das Verfahren einen schrittweisen Ablauf umfasst, einschließend einen Startschritt, bei dem die Einrichtung aktiviert wird, gefolgt von einem Warteschritt, bei dem Sensorsignale, die dem Messwert entsprechen, hinsichtlich der Stabilität gesteuert werden, anschließend gefolgt von einem Schritt, bei dem die alveoläre Alkoholkonzentration von Alkohol in Echtzeit und der Messfehler gemessen und berechnet sowie visualisiert werden, wobei die alveoläre Alkoholkonzentration von Alkohol in Echtzeit und der Messfehler während einer bestimmten Messzeit akkumulierte Messwerte sind, und einen abschließenden Schritt, der daraus folgt, dass eine akkumulierte Ungenauigkeit ein Minimum erreicht hat, wobei die akkumulierte Ungenauigkeit ein Verhältnis des akkumulierten Messfehlers zur akkumulierten alveolären Alkoholkonzentration von Alkohol in Echtzeit ist.

2.  Verfahren nach Anspruch 1, wobei die Visualisierung symbolischer oder numerischer Art ist und an die Testperson oder den Bediener gerichtet ist.

3.  Verfahren nach Anspruch 1, wobei die Alkoholbestimmung auf einer physikalischen Eigenschaft, insbesondere der Infrarotabsorption, basiert und der Messfehler nach der Minimierung im Wesentlicher stochastischer Art ist.

4.  Einrichtung zur Bestimmung der Alkoholkonzentration gemäß dem Verfahren nach Anspruch 1, umfassend mindestens

    einen ersten Sensor, dessen Ausgangssignal eine monotone Funktion der Alkoholkonzentration ist,
    einen zweiten Sensor, dessen Ausgangssignal eine monotone Funktion der Verdünnung der ausgeatmeten Luft ist, wobei die Reaktionszeit des ersten und zweiten Sensors 0,5 Sekunden nicht überschreitet,
    eine elektronische Einheit, einschließend einen Mikroprozessor, zur Ausführung eines in einem Speicher gespeicherten Programms zur Verarbeitung der von den Sensoren erzeugten Signalen, einschließend die Berechnung des Messwerts, des Messfehlers und der Bestimmung der Alkoholkonzentration,
    eine Präsentationseinheit zur audiovisuellen oder haptischen Visualisierung der Bestimmung, Messwert in Echtzeit während der laufenden Messung, wobei die Einrichtung so angeordnet ist, dass sie einen schrittweisen Ablauf durchführt, einschließend einen Startzustand, bei dem die Einrichtung aktiviert wird, gefolgt von einem Wartezustand, bei dem Sensorsignale, die dem Messwert entsprechen, hinsichtlich der Stabilität gesteuert werden, anschließend gefolgt von einem Zustand, bei dem die alveoläre Alkoholkonzentration von Alkohol in Echtzeit und der Messfehler gemessen und berechnet sowie visualisiert werden, wobei die alveoläre Alkoholkonzentration von Alkohol in Echtzeit und der Messfehler während einer bestimmten Messzeit akkumulierte Messwerte sind, und einen abschließenden Zustand, der daraus folgt, dass eine akkumulierte Ungenauigkeit ein Minimum erreicht hat, wobei die akkumulierte Ungenauigkeit ein Verhältnis des akkumulierten Messfehlers zur akkumulierten alveolären Alkoholkonzentration von Alkohol in Echtzeit ist.

**5.** Einrichtung nach Anspruch 4, wobei sich die Einrichtung in einem Gehäuse mit Außenabmessungen, die 120 x 120 x 30 mm nicht überschreiten, befindet, einschließend eine Vorrichtung für das gesteuerte Öffnen des Gehäuses zur Aufnahme der ausgeatmeten Luft.

**6.** Einrichtung nach Anspruch 4, umfassend mindestens einen Sensor zur Messung der Temperatur oder der Strömungsgeschwindigkeit der ausgeatmeten Luft, wobei die Reaktionszeit des Sensors 0,5 Sekunden nicht überschreitet.

**7.** Einrichtung nach Anspruch 4, umfassend eine Messzelle mit einer Öffnung zur Aufnahme der ausgeatmeten Luft, wobei die Messzelle mit kollimierter Infrarotstrahlung durchleuchtet wird, und ein Mittel für die aktive Strömung.

**Revendications**

**1.** Procédé pour la détermination d'une concentration d'alcool dans de l'air expiré à partir d'un échantillon d'haleine délivré par une personne test soufflant vers un agencement de détection dans un appareil pour une détermination de concentration d'alcool, par un échantillonnage sans contact dudit air expiré dans lequel l'échantillon d'haleine est dilué avec l'air ambiant, comprenant une interaction entre l'appareil d'une part et la personne test ou un opérateur d'autre part, dans lequel

ladite interaction comprend une unité de présentation au sein de l'appareil présentant une valeur de mesure et une erreur de mesure de ladite détermination à ladite personne test ou audit opérateur en temps réel pendant une mesure en cours ;
ladite erreur de mesure dépend de la dilution dudit air expiré, du temps accumulé de ladite détermination, et du bruit, des interférences ou d'autres variations aléatoires de ladite valeur de mesure, ladite dilution étant déterminée par une mesure de température, de concentration de vapeur d'eau ou de dioxyde de carbone, ou d'une combinaison de ces entités, dans lequel
une adaptation de l'exactitude et du laps de temps de ladite détermination à l'objectif ou l'exigence du moment est effectuée de manière interactive entre l'appareil et la personne test ou l'opérateur en ce que la personne test ou l'opérateur choisit de terminer la détermination ou de poursuivre la détermination, dans lequel le laps de temps fait référence au temps requis pour réaliser la détermination et dans lequel le temps de mesure prolongé et la compensation d'une possible dilution dudit air expiré ont pour but d'augmenter ladite exactitude, et dans lequel
l'unité de présentation fournit une indication de la valeur de mesure et de l'erreur de mesure au moyen d'une indication audiovisuelle ou haptique à partir d'une unité de présentation au sein dudit appareil,
le procédé comprenant une procédure par étapes, incluant une étape de départ lors de laquelle ledit appareil est activé, suivie par une étape d'attente lors de laquelle des signaux de capteur correspondant à ladite valeur de mesure sont contrôlés par rapport à la stabilité, suivie ultérieurement par une étape de mesure et de calcul lors de laquelle une concentration alvéolaire d'alcool en temps réel et une erreur de mesure sont réalisées et visualisées, dans lequel la concentration alvéolaire d'alcool en temps réel et l'erreur de mesure sont des valeurs mesurées accumulées pendant un certain temps de mesure et une étape finale découlant du fait qu'une inexactitude accumulée a atteint un minimum, l'inexactitude accumulée étant un rapport entre l'erreur de mesure accumulée et la concentration alvéolaire d'alcool en temps réel accumulée.

**2.** Procédé selon la revendication 1, dans lequel ladite visualisation présente un caractère symbolique ou numérique, et est dirigée vers ladite personne test ou ledit opérateur.

**3.** Procédé selon la revendication 1, dans lequel ladite détermination d'alcool est basée sur une propriété physique, qui est en particulier une absorption infrarouge, et en ce que ladite erreur de mesure après minimisation présente essentiellement un caractère stochastique.

**4.** Appareil pour la détermination d'une concentration d'alcool selon le procédé selon la revendication 1, comprenant au moins

un premier capteur dont le signal de sortie est une fonction monotone de ladite concentration d'alcool,
un second capteur dont le signal de sortie est une fonction monotone de la dilution dudit air expiré, selon lequel le temps de réponse desdits premier et second capteurs n'excède pas 0,5 seconde,
une unité électronique incluant un microprocesseur, pour l'exécution d'un programme stocké dans une mémoire pour traiter lesdits signaux générés par lesdits capteurs incluant le calcul de la valeur de mesure, de l'erreur de mesure et ladite détermination de concentration d'alcool,

une unité de présentation pour une visualisation audiovisuelle ou haptique de ladite détermination, valeur de mesure en temps réel pendant une mesure en cours, dans lequel l'appareil est agencé pour réaliser une procédure par étapes, incluant une condition de départ, lors de laquelle ledit appareil est activé, suivie par une condition d'attente lors de laquelle des signaux de capteur correspondant à ladite valeur de mesure sont contrôlés par rapport à la stabilité, suivie ensuite par une condition de mesure et de calcul lors de laquelle une concentration alvéolaire d'alcool en temps réel et une erreur de mesure sont réalisées et visualisées, dans lequel la concentration alvéolaire d'alcool en temps réel et l'erreur de mesure sont des valeurs mesurées accumulées pendant un certain temps de mesure et une condition finale découlant du fait qu'une inexactitude accumulée a atteint un minimum, l'inexactitude accumulée étant un rapport entre l'erreur de mesure accumulée et la concentration alvéolaire d'alcool en temps réel accumulée.

5. Appareil selon la revendication 4, dans lequel ledit appareil est incorporé dans un boîtier avec des dimensions externes n'excédant pas 120 x 120 x 30 mm, incluant un dispositif pour une ouverture commandée dudit boîtier pour recevoir ledit air expiré.

6. Appareil selon la revendication 4, comprenant au moins un capteur pour la mesure desdites température ou vitesse d'écoulement dudit air expiré, selon lequel le temps de réponse dudit capteur n'excède pas 0,5 seconde.

7. Appareil selon la revendication 4, comprenant une cellule de mesure avec une ouverture pour la réception dudit air expiré, ladite cellule de mesure étant éclairée par transparence par un rayonnement infrarouge collimaté, et des moyens pour un écoulement actif.

Fig 1

Fig 2

Fig 3

41    39

32

33

37    38    42

34    35    36

40

43

0,08 ± 0,02 MG/L

44

45    31

Fig 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007016092 A1
- US 5531225 A
- US 5376555 A
- US 2004141171 A1
- US 2006153740 A1
- WO 2007046745 A1
- US 2008061238 A1